# EUROPEAN PATENT APPLICATION

(11) **EP 4 321 188 A1**
(43) Date of publication of application: **14.02.2024**
(21) Application number: 23188864.5
(22) Date of filing: 01.08.2023
(51) Int. Cl.: A61M 5/142, A61M 5/44, F16L 23/00, F16L 37/00, F04B 1/00

(54) **PUMP ASSEMBLY FOR THE TREATMENT OF INJECTION MEDICAL DEVICES**

(30) Priority: 11.08.2022 IT 202200017175
(71) Applicant: NUOVA OMPI S.r.l. Unipersonale, 35017 Piombino Dese (PD) (IT)
(72) Inventor: TACCHINI, Paolo, I-40069 Zola Predosa (BO) (IT); TONINI, Filippo, I-40134 Bologna (IT); CHINELLATO, Fabio, I-31100 Treviso (IT); CHILLON, Alberto, I-35010 San Vito di Vigonza (PD) (IT)
(74) Representative: Porta & Consulenti Associati S.p.A.

(57) **Abstract**

A pump assembly (10) for the treatment of injection medical devices comprises a pump casing (11) housing a driver connected to a pumping body (12) rotating around a rotational axis (X), a pump head (20) comprising a pump chamber (13), an inlet port (23) and a delivery port (24) in fluid connection with said pump chamber (13), a inlet manifold (25) and a delivery manifold (45). Both the inlet manifold (25) and the delivery manifold (45) comprise respectively a first tubular body (26, 46) connected to the pump head (20) and comprising an internal cavity (28, 48) facing respectively the inlet port (23) and the delivery port (24), and a first flange (29, 49) comprising an abutting surface (30, 50); a second tubular body (27, 47) having an insertion portion (36, 56) inserted into the internal cavity (28, 48) of the first tubular body (26, 46) and in fluid-tight connection respectively with the inlet port (23) and the delivery port (24), and a second flange (33, 53) provided at a position between a radially inner end and a radially outer end of the second tubular body (27, 47) and comprising an abutting surface (34, 54) sets against the abutting surface (30, 50) of the first flange (29, 49). A first clamp (40) is active on the first flange (29) and the second flange (33) of the inlet manifold (25) to press the abutting surfaces (34, 30) against to each other. A second clamp (60) is active on the first flange (49) and the second flange (53) of the delivery manifold (45) to press the abutting surfaces (54, 50) against to each other.

## Description

### Field of the invention

The present invention relates to a pump assembly for the treatment of injection medical devices of the type comprising a glass cylinder configured to receive a plunger with sliding engagement.

### Background of the invention

As is well known, injection devices generally comprising a sealing plunger in sliding engagement within a container are widely used in the medical field to deliver a drug by injection to a patient.

Such injection devices include syringes, cartridges but also auto-injectors or automatic injectors used for the subcutaneous and/or intravenous administration of drugs.

In this type of device, a first requirement that needs to be met is to have optimal sliding properties (in terms of static and dynamic friction) of the plunger within the cylinder of the injection device, e.g., the cylinder of a syringe.

Additional need that is particularly felt is to keep the sliding properties of the plunger as constant as possible over time particularly in the case of injection devices such as syringes pre-filled with a drug.

In fact, while the use of pre-filled injection devices provides greater ease of drug administration and flexibility in handling, it also means that injection devices must be stored after filling for quite a long time (weeks or months) even at very low temperatures such as to ensure stability and a longer life (shelf-life) of the drug.

To meet both requirements, injection medical devices are treated using a coating composition, typically silicone oil-based, to coat the inner surface of both the syringe body and the plunger.

The silicone oil-based coating composition is sprayed through a nozzle that is fed by a pump assembly for treating a medical device for injection, on the inner surface of the syringe and possibly on the plunger.

Specifically, pump assemblies for the treatment of injection medical devices typically include a positive displacement pump having a casing, a pump head housing a rotary reciprocating piston, an inlet conduit, and a delivery conduit. The inlet conduit includes a first threaded end screwed, with a gasket seal interposed, into an inlet port formed in the pump head and a second end, also threaded, configured to receive a conduit connected to a reservoir for the coating composition. The delivery conduit includes a first threaded end screwed, with the interposition of a sealing gasket, into a delivery port formed in the pump head and a second end, also threaded, configured to receive a conduit connected to one or more dispensing nozzles for the coating composition.

### Summary of the invention

The Applicant has noted that various silicone oil-based formulations are currently used as coating composition in an attempt to improve the homogeneity of the coating layer, the lubricating properties of the coating layer, and to keep the coating layer as thin as possible.

The Applicant has noted that as the specifications of the medical devices for injection change, such as storage condition, injection rate, or the type of substance with which the device is filled, different silicone oil formulations may be used to coat the medical device for injection.

The Applicant also has noted that when it is necessary to change the specifications of the injection medical devices in a production line of injection medical devices, it must be ensured that the new coating composition to be used is not contaminated by the previously used coating composition.

However, the Applicant has noted that this requires the use of a different pump assembly to process the new medical devices for injection, or a complete disassembly and flushing of the pump assembly already in use to process the new medical devices for injection.

The Applicant has noted that in case a different pump assembly is used, it is necessary to have a large number of pump assemblies for the treatment of injection medical devices in which, from time to time, only one pump assembly is in use, resulting in high injection medical devices manufacturing cost necessary to produce and store ready-to-use a large number of pump assemblies for the treatment of injection medical devices.

The Applicant also noted that in case the pump assembly already in use is completely disassembled and flushed, a non-negligible downtime is required for complete disassembly and flushing operations, so resulting in high injection medical devices manufacturing cost.

In fact, the Applicant for example has noted that the reassembly of the inlet conduit and of the delivery conduit requires careful tightening of the first end of the inlet conduit and of the delivery conduit to ensure proper sealing of the respective gaskets located between the inlet conduit and the inlet port and between the delivery conduit and the delivery port. These operations require, in addition to non-negligible time, the employment of skilled labor.

The Applicant has perceived that manufacturing cost of injection medical devices could be reduced by proposing a pump assembly for the treatment of injection medical devices that allows quick disassembly and reassembly of components that come in contact with the coating composition.

The Applicant has found that the inlet conduit and delivery conduit could be arranged with respective first tubular bodies stably connected to the inlet port and to the delivery port and respective second tubular bodies inserted within the first tubular bodies so as to fluid-tightly contact the inlet port and delivery port.

The Applicant has found that by equipping the first tubular bodies and the second tubular bodies with respective abutting surfaces and holding the abutting surfaces of the second tubular bodies in contact against the respective abutting surfaces of the first tubular bodies, it would be possible to assemble the inlet conduit and the outlet conduit by simply inserting the second tubular bodies into the first tubular bodies and holding the abutting surfaces against to each other, and it would be possible to disassemble the inlet conduit and the outlet conduit by simply removing the second tubular bodies from the first tubular bodies by moving away the abutting surfaces from each other.

The Applicant has found that this avoids calibrated tightening of the inlet conduit into the inlet port, since by appropriately choosing the distance between the abutting surfaces of the tubular bodies of the inlet conduit and the inlet port, it is possible to ensure that the second tubular body reaches the inlet port and fluid-tight fits on the latter, possibly by interposing a sealing gasket.

Similarly, the Applicant has found that this avoids calibrated tightening of the delivery conduit in the delivery port, since by appropriately choosing the distance between the abutting surfaces of the tubular bodies of the delivery conduit and the delivery port, it is possible to ensure that the second tubular body reaches the delivery port and fluid-tightly fits on the latter, possibly by interposition of a sealing gasket.

In this way, disassembly of the inlet conduit and of the delivery conduit and subsequent reassembly operations of the inlet conduit and of the delivery conduit to carry out cleaning operations necessary for changing the coating composition are simplified, downtime is reduced, and highly skilled labor is not required, decreasing the production costs of medical injection devices.

Therefore, the present invention relates to a pump assembly for the treatment of injection medical devices comprising:
a pump casing housing a driver connected to a pumping body rotating around a rotational axis;
a pump head comprising a pump chamber wherein the pumping body acts into said pump chamber for pressurizing a coating composition,
an inlet port for a coating composition and a delivery port for said coating composition, wherein said inlet port and said delivery port are in fluid connection with said pump chamber;
an inlet manifold for entering said coating composition into said inlet port and a delivery manifold for exiting said coating composition from said delivery port;
wherein both the inlet manifold and the delivery manifold comprise respectively:
   a first tubular body connected to the pump head and comprising an internal cavity facing respectively the inlet port and the delivery port, and a first flange comprising an abutting surface;
   a second tubular body having an insertion portion inserted into the internal cavity of the first tubular body and in fluid-tight connection respectively with the inlet port and the delivery port, and a second flange provided at a position between a radially inner end and a radially outer end of the second tubular body and comprising an abutting surface set against the abutting surface of the first flange;
   the pump assembly further comprising a first clamp active on the first flange and the second flange of the inlet manifold to press the abutting surface of the second flange against the abutting surface of the first flange and a second clamp active on the first flange and the second flange of the delivery manifold to press the abutting surface of the second flange against the abutting surface of the first flange.

The terms "radial" and "axial" are used with reference, respectively, to a direction parallel to the rotational axis of the pumping body and to a direction contained in a plane perpendicular to the rotational axis of the pumping body and intersecting the rotational axis of the pumping body.

The expressions "radially inner" and "radially outer" indicate a position respectively closer to, and further from, the rotation axis of the pumping body.

The terms "circumferential" and "circumferentially" are used with reference to circular directions centered on the rotation axis of the pumping body and contained in a plane perpendicular to the rotation axis of the pumping body.

The expression "circumferentially adjacent" when referred to two components of the pump assembly is used to indicate that a first component precedes or follows a second component along a circumferential direction.

The present invention may comprise one or more of the following preferred features, which may be combined with each other as desired according to specific needs.

Preferably, the insertion portion of the second tubular body of the inlet manifold extends from the abutting surface of the first flange of the first tubular body of the inlet manifold to a gasket seal located at the inlet port.

Preferably, the gasket seal located at the inlet port is partially inserted into a ring groove provided at a radially inner end of the insertion portion of the second tubular body of the inlet manifold.

Preferably, a distance in a radial direction between the abutting surface of the first flange of the first tubular body of the inlet manifold and the inlet port is substantially equal to the sum of length of the insertion portion and of a length of a portion of the gasket seal extending radially beyond the ring groove.

Preferably, the length of the insertion portion is measured from the abutting surface to the radially inner end of the second tubular body of the inlet manifold.

Preferably, the ring groove holds the gasket seal at the radially inner end of the insertion portion of the second tubular body of the inlet manifold.

Preferably, the insertion portion of the second tubular body of the delivery manifold extends from the abutting surface of the first flange of the first tubular body of the delivery manifold to a gasket seal located at the delivery port.

Preferably, the gasket seal located at the delivery port is partially inserted into a ring groove provided at a radially inner end of the insertion portion of the second tubular body of the delivery manifold.

Preferably, a distance in a radial direction between the abutting surface of first flange of the first tubular body of the delivery manifold and the delivery port is substantially equal to the sum of a length of the insertion portion and a length of a portion of the gasket seal extending radially beyond the ring groove.

Preferably, the length of the insertion portion is measured from the abutting surface to the radially inner end of the second tubular body of the delivery manifold.

Preferably, the ring groove holds the gasket seal at the radially inner end of the insertion portion of the second tubular body of the delivery manifold.

Preferably, the first tubular body of the inlet manifold is integral with the pump head.

Preferably, the first tubular body of the delivery manifold is integral with the pump head.

Preferably, the first flange of the first tubular body of the inlet manifold comprises a conical surface opposite to the abutting surface.

Preferably, the abutting surface of the first flange is radially outer with respect to the conical surface of the first flange.

Preferably, the second flange of the second tubular body of the inlet manifold comprises a conical surface opposite to the abutting surface.

Preferably, the abutting surface of the second flange is radially inner with respect to the conical surface of the second flange.

Preferably, the first clamp comprises a first conical contact surface and a second conical contact surface.

Preferably, the first clamp comprises a clamping screw for slidingly coupling the first conical contact surface and the second conical contact surface to the respective conical surfaces of the first flange and of the second flange.

By tightening the clamping screw, the first conical contact surface and the second conical contact surface slide along the conical surfaces of the first flange and of the second flange so as to press the first flange against the second flange.

When the first flange is pressed against the second flange, the abutting surface of the first tubular body and the abutting surface of the second tubular body are pressed against each other, so providing a fluid tightness between the first flange and the second flange.

Preferably, the first conical contact surface and a second conical contact surface of the first clamp face to each other in a radial direction.

Preferably, the first conical contact surface of the first clamp contacts the conical surface of the first flange.

Preferably, the second conical contact surface of the first clamp contacts the conical surface of the second flange.

In a preferred embodiment, the first clamp comprises a first clamp portion and a second clamp portion hinged to each other, along a hinge axis radially oriented, at respective first end portions.

Preferably, the first clamp portion and a second clamp portion comprise a respective second end portion circumferentially opposite to the respective first end portion.

Preferably, a first half of the first conical contact surface and a first half of the second conical contact surface are provided on the first clamp portion and a second half of the first conical contact surface and a second half of the second conical contact surface are provided on the second clamp portion.

Preferably, the clamping screw is active at the second end portions of the first clamp portion and of the second clamp portion. By tightening the clamping screw the second end portions of the first clamp portion and of the second clamp portion move close to each other while the first clamp portion and the second clamp portion rotates around the hinge axis.

In a preferred embodiment, the clamping screw is a butterfly screw that does not require any tool for being screwed and unscrewed.

Preferably, the first flange of the first tubular body of the delivery manifold comprises a conical surface opposite to the abutting surface.

Preferably, the abutting surface of the first flange is radially outer with respect to the conical surface of the first flange.

Preferably, the second flange of the second tubular body of the delivery manifold comprises a conical surface opposite to the abutting surface.

Preferably, the abutting surface of the second flange is radially inner with respect to the conical surface of the second flange.

Preferably, the second clamp comprises a first conical contact surface and a second conical contact surface.

Preferably, the second clamp comprises a clamping screw for slidingly coupling the first conical contact surface and the second conical contact surface to the respective conical surfaces of the first flange and of the second flange.

By tightening the clamping screw, the first conical contact surface and the second conical contact surface slide along the conical surfaces of the first flange and of the second flange so as to press the first flange against the second flange.

When the first flange is pressed against the second flange, the abutting surface of the first tubular body and the abutting surface of the second tubular body are pressed against each other, so providing a fluid tightness between the first flange and the second flange.

Preferably, the first conical contact surface and a second conical contact surface of the second clamp face to each other in a radial direction.

Preferably, the first conical contact surface of the second clamp contacts the conical surface of the first flange.

Preferably, the second conical contact surface of the second clamp contacts the conical surface of the second flange.

In a preferred embodiment, the second clamp comprises a first clamp portion and a second clamp portion hinged to each other, along a hinge axis radially oriented, at respective first end portions.

Preferably, the first clamp portion and a second clamp portion comprise a respective second end portion circumferentially opposite to the respective first end portion.

Preferably, a first half of the first conical contact surface and a first half of the second conical contact surface are provided on the first clamp portion and a second half of the first conical contact surface and a second half of the second conical contact surface are provided on the second clamp portion.

Preferably, the clamping screw is active at the second end portions of the first clamp portion and of the second clamp portion. By tightening the clamping screw the second end portions of the first clamp portion and of the second clamp portion move close to each other while the first clamp portion and the second clamp portion rotates around the hinge axis.

In a preferred embodiment, the clamping screw is a butterfly screw that does not require any tool for being screwed and unscrewed.

Preferably, the pump assembly comprises a first electric heater and a second electric heater active in the pump head at the pump chamber and configured to heat said pump head.

In this regard, the Applicant has noted that the different silicone oil-based formulations suitable as coating composition may have, among other characteristics, different kinematic viscosity values at room temperature that can range from rather low values (around 1000 cSt) to high values (around 10000 cSt and even higher).

The Applicant has noted that silicone oil-based formulations having different kinematic viscosity values at room temperature require to be heated to different temperatures or ranges of temperatures in order to be adequately delivered by the pump assembly to the nozzles that spray the coating composition on the medical device for injection.

Specifically, the Applicant has noted that silicone oil-based formulations that have high kinematic viscosity values at room temperature must be heated to higher temperatures than silicone oil-based formulations that have low kinematic viscosity values.

In fact, the Applicant has noted that in order to evenly distribute the coating composition on the inner surface of the syringe and possibly on the plunger, the nozzles need to atomize the lubricating substance by making an aerosol in which particles of a substance in a gaseous state (e.g., air) are suspended in the coating composition. To ensure adequate creation of such an aerosol, the coating composition must be heated to a predetermined temperature or maintained within a predetermined temperature range.

The Applicant has perceived that by providing the pump assembly with at least a first electric heater and a second electric heater active in the pump head at the pump chamber, it is possible to use silicone oil-based formulations having different kinematic viscosity values at room temperature using the same pump assembly.

The Applicant has indeed perceived that once the pump assembly has been dismounted, cleaned and remounted for processing injection medical devices with a different specification, the pump head and the pump chamber can be heated at a different temperature or range of temperature allowing to use a silicone oil-based formulation having a different kinematic viscosity value at room temperature.

The electric heaters allow to maintain the pump head and the pump chamber provided within the pump head at a predetermined range of temperature.

The Applicant has found that the provision of two electric heaters can enhance a uniform distribution of temperature within the pump head.

In a preferred embodiment, the first electric heater and the second electric heater are spaced apart from each other in a circumferential direction by an angle comprised between 90° and 180°.

Preferably, the first electric heater and the second electric heater are spaced apart in the circumferential direction by an angle of about 180°.

The circumferential spacing between the first electric heater and the second electric heater contributes in evenly heating the pump head.

Preferably, the pump assembly comprises a third electric heater active in the pump head at the pump chamber.

Preferably, the third electric heater is configured to heat said pump head, cooperating with the first electric heater and the second electric heater in evenly heating the pump head.

Preferably, the pump assembly comprises a thermal probe active in the pump head at the pump chamber.

Preferably, said thermal probe is configured to sense a temperature of the pump head at the pump chamber.

Preferably, said third electric heater is circumferentially interposed between said first electric heater and said second electric heater.

Preferably, said second electric heater is circumferentially interposed between said third electric heater and said thermal probe.

Preferably, said thermal probe is circumferentially interposed between said first electric heater and said second electric heater.

Accordingly, in a preferred embodiment the first electric heater, the second electric heater, the third electric heater and the thermal probe are placed on the head pump so that, by proceeding along a circumferential direction the third electric heater follows the first electric heater, the second electric heater follows the third electric heater, the thermal probe follows the second electric heater and the first electric heater follows the thermal probe.

In order to maximize the uniform distribution of the temperature within the pump head and within the pump chamber, preferably any two circumferentially adjacent elements selected from group consisting of: first electric heater, second electric heater, third electric heater and thermal probe, are spaced apart from each other in the circumferential direction by angles comprised between 60° and 120°.

In a preferred embodiment, the first electric heater and the third electric heater are spaced apart in the circumferential direction by an angle of about 60°.

Preferably, the third electric heater and the second electric heater are spaced apart in the circumferential direction by an angle of about 120°.

Preferably, the second electric heater and the thermal probe are spaced apart in the circumferential direction by an angle of about 60°.

Preferably, the thermal probe and the first electric heater are spaced apart in the circumferential direction by an angle of about 120°.

Preferably, the first electric heater and the second electric heater are held in place through a holding ring.

In the preferred embodiment comprising the third electric heater and the thermal probe, the third electric heater and the thermal probe are held in place through the holding ring too.

Preferably, said holding ring is mechanically connected to the pump head in a position axially opposite to the pump casing.

Preferably, said holding ring is mechanically connected to the pump head by butterfly screws that do not require any tool for being screwed and unscrewed.

By unscrewing the butterfly screws, the holding ring along with the first electric heater, the second electric heater, the third electric heater and the thermal probe can be simultaneously dismounted from the head pump.

Preferably, said butterfly screws are screwed into the head pump.

Preferably, there are provided two butterfly screws spaced apart in the circumferential direction by an angle of about 180°.

In a preferred embodiment, one butterfly screw is circumferentially interposed between the second electric heater and the third electric heater and the other butterfly screw is circumferentially interposed between the thermal probe and the first electric heater.

Preferably, said thermal probe, said first electric heater, said second electric heater and said third electric heater are in signal communication with a control unit.

Preferably, said control unit is configured to maintain the pump head temperature within a predetermined temperature range.

Preferably, said control unit is configured to receive a first input data representative of a settled temperature or of a settled range of temperature and to generate a second input data suitable for activating said first electric heater, said second electric heater and said third electric heater (when present).

Preferably, said first input data is generated by a user interface configured for being operated by a user.

Preferably, said control unit is configured to receive a third input data from said thermal probe representative of a temperature sensed by the thermal probe.

Preferably, said control unit is configured to compare said first input data and said third input data and to generate said second input data when the sensed temperature is lower than the settled temperature or of a minimum value of the settled range of temperature.

Preferably, said control unit is configured to compare said first input data and said third input data and to generate a fourth input data when the sensed temperature is higher than the settled temperature or of a maximum value of the settled range of temperature.

Preferably, said fourth input data is suitable for deactivating said first electric heater, said second electric heater and said third electric heater (when present).

In a preferred embodiment, the pump assembly comprises a connection interface placed between said pump head and said pump casing.

Preferably, said connection interface is mechanically connected to said pump casing through a first plurality of bolts and said pump head is connected to said connection interface through a second plurality of bolts different from said first plurality of bolts.

The connection interface allows to dismount the head pump from the pump casing by mechanically disconnecting the connection interface from the pump casing. The Applicant has perceived that this allows to configure the connection interface so that the first plurality of bolts are easily accessible during the mounting and dismounting operations. In fact, during this operations the pump head can remain mechanically connected to the connection interface. Moreover, the first plurality of bolts only acts between the connection interface and the pump casing and the connection interface is not affected by all the design limitations that affect the head pump (that must be designed for containing the pump chamber, the inlet port, the delivery port, for connecting the inlet manifold to the inlet port and for connecting the delivery manifold to the delivery port), so allowing the first plurality of bolts to be actually easily accessible during the mounting and dismounting operations of the pump head.

In this respect, preferably, said connection interface comprises a through slot for each bolt of said first plurality of bolts, wherein said through slot comprises a first portion configured to be contacted by a bolt head to axially retain the connection interface on the pump casing and a second portion, circumferentially adjacent to the first portion, configured to be traversed by the bolt head.

Thus, the connection interface can be dismounted from the pump casing by loosen the first plurality of bolts (without the need to completely remove the first plurality of bolts), rotating the connection interface along with the head pump so as to align the bolt heads to the second portion of the through slots and then extracting the connection interface along with the head pump while leaving the first plurality of bolts partially screwed into the pump casing.

Preferably, the pump chamber is closed at an axial end by a window provided in the head pump, so allowing to see the interior of the pump chamber during the pump assembly functioning.

Preferably, the pump chamber and the pumping body define a volumetric pump.

Preferably, such volumetric pump is a rotary reciprocating piston pump.

In a preferred embodiment, the pumping body is a piston rotating within the pump chamber around said rotational axis and reciprocating within the pump chamber.

### Brief description of the drawings

Further features and advantages of the invention will become clearer from the following description of a preferred embodiment, made with reference to the attached drawings. The drawings are schematic and made for illustrative and non-limiting purposes.

In the drawings:
- Figure 1 illustrates a perspective view of a pump assembly for the treatment of injection medical devices according to the present invention;
- Figure 2 illustrates a lateral view of the pump assembly of figure 1;
- Figure 3 is a section view along the plan III-III of the pump assembly of figure 2 with some components removed for better illustrating other components;
- Figures 4 and 5 are sectional views according to the sectional view of figure 3 of an inlet manifold and of a delivery manifold of the pump assembly according to the present invention;
- Figure 6 is a front view of the pump assembly of figure 1 with some components removed for better illustrating other components;
- Figure 7 is a further perspective view of the pump assembly of figure 1 with some components removed for better illustrating other components;
- Figure 8 is a partially exploded perspective view of the pump assembly of figure 1 with some components removed for better illustrating other components; and
- Figure 9 is a diagrammatic view of some components of the pump assembly according to the present invention.

### Detailed description of the preferred embodiment

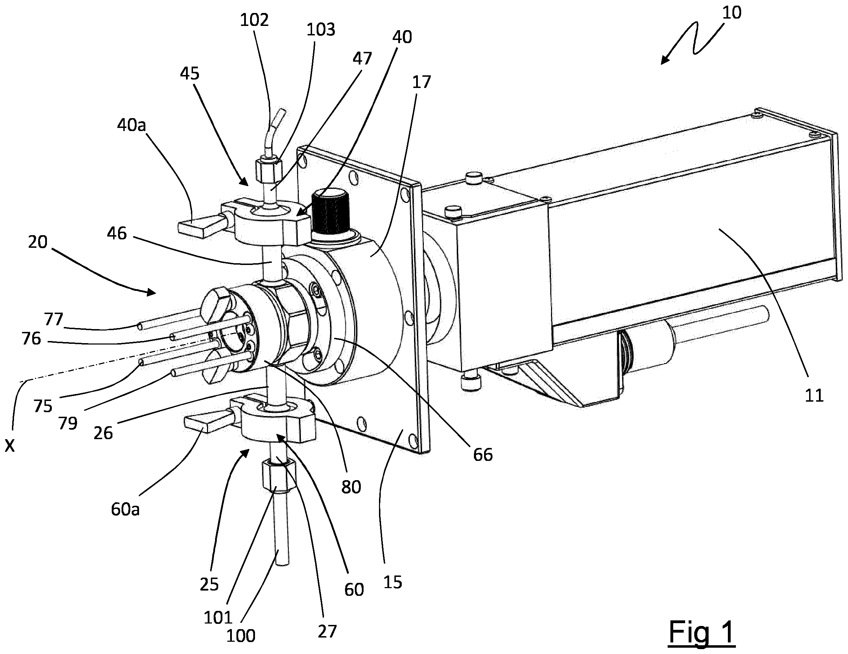

A pump assembly for the treatment of injection medical devices according to a preferred embodiment of the invention is referred to by reference number 10 in Figure 1.

The pump assembly 10 comprises a pump casing 11 containing a driver (not illustrated), for example an electric motor, and related devices for actuating the driver.

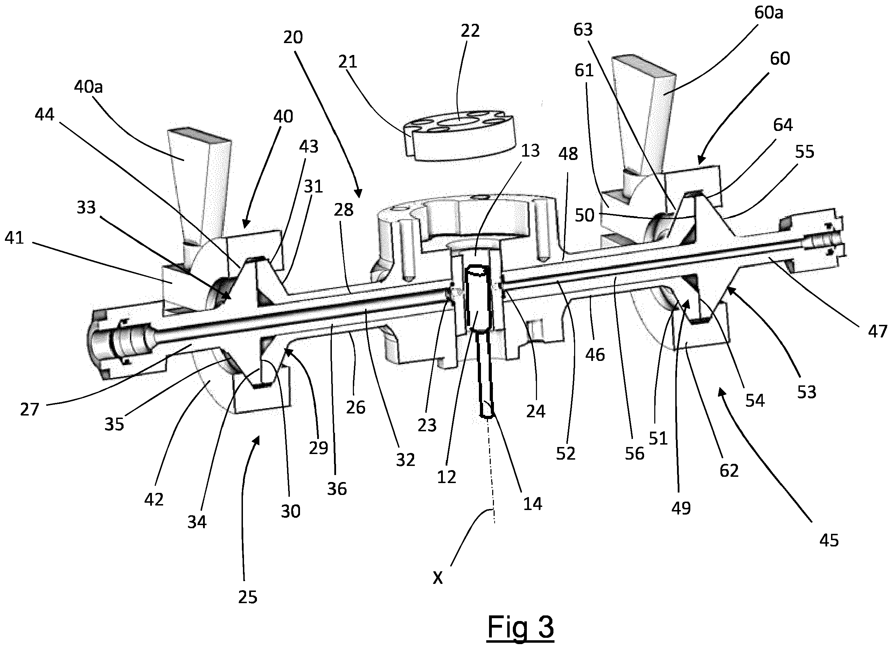

The driver is configured for actuating a pumping body 12 (schematically illustrated in figure 3) within a pump chamber 13. The driver and the pumping body 12 are connected to each other by a pump shaft 14 (schematically illustrated in figure 3).

In the preferred embodiment of the invention, the pumping body 12 is a piston rotating within the pump chamber 13 around a rotational axis X and reciprocating within the pump chamber 13 along the rotational axis X. The pump chamber 13 and the pumping body 12 realizes a single valveless reversible positive displacement pump, specifically a rotary reciprocating piston pump.

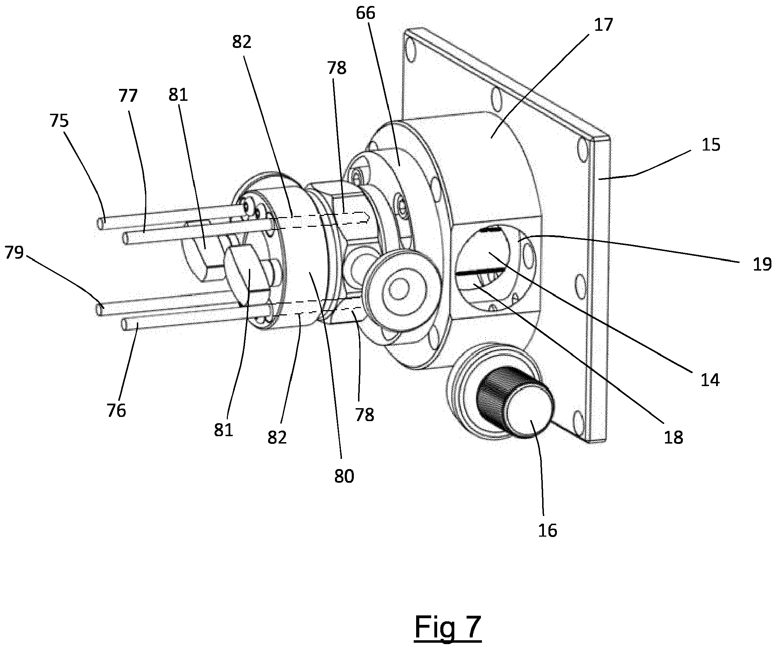

The pump casing 11 comprises a connecting plate 15 for mounting the pump assembly 10 on a frame of a production line. The pump casing 11 further comprises an inspection bolt 16 and an inspection casing 17 placed at an axial end of the pump casing 11. The inspection casing 17 is placed axially opposite to the driver with respect to the connecting plate 15. The inspection casing 17 defines an inspection cavity 18 crossed by the pump shaft 14. The inspection bolt 16 is configured for being screwed and unscrewed in an inspection aperture 19 of the inspection casing 17 (figure 7). By unscrewing the inspection bolt 16, the inspection cavity 18 can be accessed and the pump shaft 14 can be accessed for maintenance or for cleaning operation.

The pump assembly 10 comprises a pump head 20 operatively connected to the pump casing 11.

The pump head 20 comprises the pump chamber 13 that has a cylindrical shape and that receives the pumping body 12. The pump chamber 13 is open at an axial end facing the pump casing 11 and is closed at an opposite axial end by a cap 21 (illustrated in an exploded view in figure 3). The cap 21 is a part of the pump head 20 and comprises a window 22 facing the pump chamber 13. The cap 21 can be dismounted from the pump chamber 13 for accessing the pump chamber 13 during deep cleaning.

The pump chamber 13 comprises an inlet port 23 and a delivery port 24. The inlet port 23 allows the access of a coating composition to be pressurized into the pump chamber 13 and the delivery port 24 allows the exit of the coating composition pressurized from the pump chamber 13. The inlet port 23 and the delivery port 24 are provided at opposite circumferential position on the pump chamber 13. The inlet port 23 and the delivery port 24 are spaced apart of about 180° in a circumferential direction.

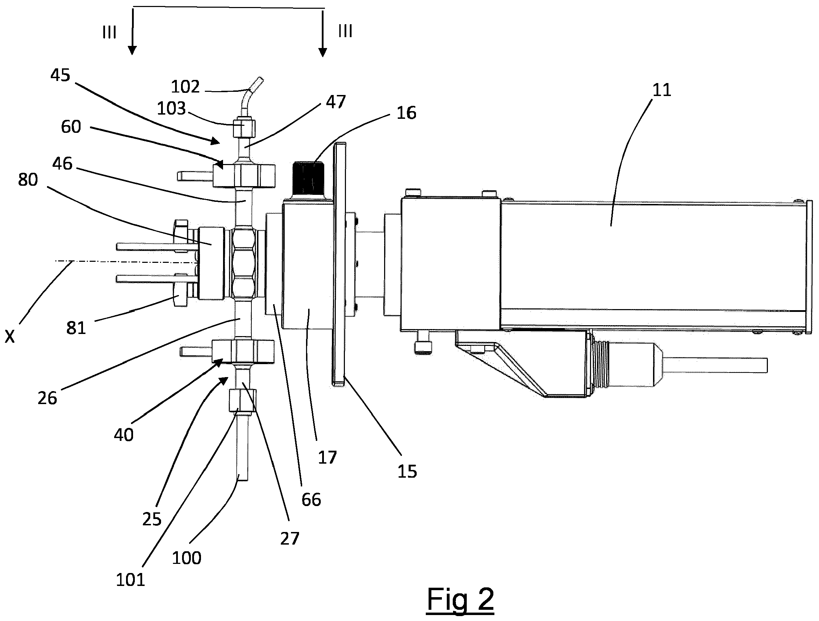

For allowing the coating composition to reach the inlet port 23 the pump head 20 comprises an inlet manifold 25 that is in fluid communication with the inlet port 23 and that can be fluidly connected to a reservoir (not illustrated) through a inlet pipe 100 (partially illustrated in figures 1 and 2).

The inlet manifold 25 is substantially realized in two dismountable parts. Particularly, the inlet manifold 25 comprises a first tubular body 26 and a second tubular body 27.

The first tubular body 26 is connected to the pump head 20 preferably permanently. In the preferred embodiment, the first tubular body 26 is integral with the pump head 20. The first tubular body 26 comprises an internal cavity 28 that extends along the whole first tubular body 26 and that is in fluid communication with the inlet port 23.

At an outer radial end of the first tubular body 26, there is provided a first flange 29 having an abutting surface 30. The first flange 29 is integral with the first tubular body 26. The abutting surface 30 is a planar surface of annular shape. At the abutting surface 30 the internal cavity 28 is open in a radially outer direction. The first flange 29 further comprises a conical surface 31 radially opposite to the abutting surface 30. The first flange 29 is tapered at the conical surface 31 so as to link the abutting surface 30 to a lateral wall of the first tubular body. The first flange 29 is tapered at the conical surface 31 so as to progressively increase its transversal section in a radial direction directed toward the abutting surface 30. The abutting surface 30 is radially outer with respect to the conical surface 31 of the first flange 29. The conical surface 31 of the first flange 29 is radially interposed between the inlet port 23 and the abutting surface 30.

The second tubular body 27 comprises an internal cavity 32 that extends along the whole second tubular body 27 from a radially inner end to a radially outer end. The radially outer end of the second tubular body 27 can be fluidly connected to a reservoir (not illustrated) through an inlet pipe 100 for example by a compression fitting 101.

The second tubular body 27 comprises a second flange 33 having an abutting surface 34. The second flange 33 is integral with the second tubular body 27. The second flange 33 is provided along the second tubular body 27 at a position between the radially inner end and the radially outer end of the second tubular body 27. The abutting surface 34 is a planar surface of annular shape and presents the same dimension of the abutting surface 30 of the first flange 29. The abutting surface 34 is countershaped to the abutting surface 30 of the first flange 29. The second flange 33 along with the abutting surface 34 is crossed by the internal cavity 32. The second flange 33 further comprises a conical surface 35 radially opposite to the abutting surface 34. The second flange 33 is tapered at the conical surface 35 so as to link the abutting surface 34 to a lateral wall of the second tubular body.

The second flange 33 is tapered at the conical surface 35 so as to progressively increase its transversal section in a radial direction directed toward the abutting surface 34. The abutting surface 34 is radially inner with respect to the conical surface 35 of the second flange 33. The abutting surface 34 is radially interposed between the inlet port 23 and the conical surface 35 of the second flange 33.

The abutting surface 34 of the second flange 33 is coupled to the abutting surface 30 of the first flange 29.

The second tubular body comprises an insertion portion 36 slidingly inserted into the internal cavity 28 of the first tubular body 26 and in fluid-tight connection with the inlet port 23. The insertion portion 36 has a transversal dimension (namely a dimension along a plane perpendicular to the redial extension of the insertion portion 36) that is slightly smaller that a transversal dimension of the internal cavity 28 of the first tubular body 26.

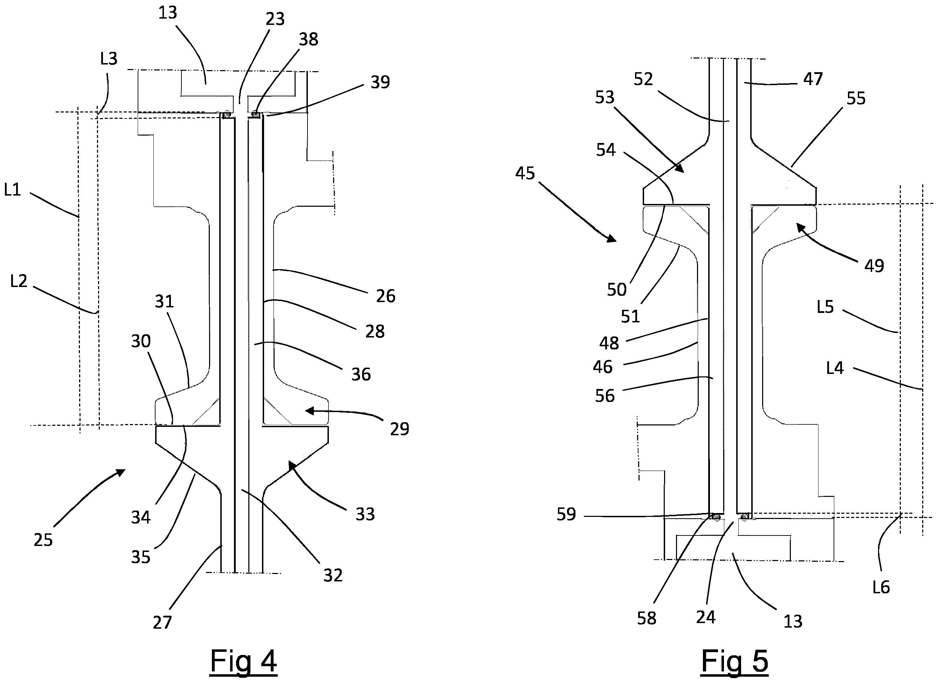

The insertion portion 36 substantially extends from the abutting surface 34 to the inlet port 23. Particularly, a gasket seal 38 is interposed between the insertion portion 36 and the inlet port 23 so as to fluidly couple the internal cavity 32 to the inlet port 23. The gasket seal 38 is partially inserted into a ring groove 39 (figure 4) provided at a radially inner end of the second tubular body 27 that coincides with a radially inner end of the insertion portion 36. The ring groove 39 holds the gasket seal 38 to the second tubular body 27.

As schematically illustrated in figure 4, a distance L1 in a radial direction between the abutting surface 30 of the first flange 29 and the inlet port 23 is substantially equal to the sum of length L2 of the insertion portion 36 and a length L3 of a portion of the gasket seal 38 extending radially beyond the ring groove 39. The length L2 of the insertion portion 36 is measured from the abutting surface 34 of the second flange 33 and the inner end of the insertion portion 36.

In order to ensure a fluid tight coupling between the abutment surface 30 of the first flange 29 and the abutting surface 34 of the second flange 33 and thus a fluid tight coupling between the internal cavities 28, 32 of the first tubular body 26 and of the second tubular body 27, the abutment surface 30 of the first flange 29 and the abutting surface 34 of the second flange 33 are pressed to each other.

In this connection, the pump assembly 10 comprises a first clamp 40 active on the first flange 29 and on the second flange 33.

The first clamp 40 comprises a first clamp portion 41 and a second clamp portion 42 hinged to each other along a hinge axis radially oriented. The first clamp 40 comprises a first conical contact surface 43 and a second conical contact surface 44.

The first conical contact surface 43 extends on both the first clamp portion 41 and a second clamp portion 42, as well as the second conical contact surface 44 extends on both the first clamp portion 41 and a second clamp portion 42. In other words, the first clamp portion 41 comprises a first part of the first conical contact surface 43 and of the second conical contact surface 44 and the second clamp portion 42 comprises a second part of the first conical contact surface 43 and of the second conical contact surface 44.

The first clamp 40 is configured for clamping the first flange 29 and the second flange 33 to each other so as to couple the first tubular body 26 to the second tubular body 27.

To this end, the first conical contact surface 43 faces the conical surface 31 of the first flange 29 and the second conical contact surface 44 faces the conical surface 35 of the second flange 33. The first conical contact surface 43 is countershaped to the conical surface 31 of the first flange 29 and the second conical contact surface 44 is countershaped the conical surface 35 of the second flange 33.

The first clamp portion 41 and a second clamp portion 42 can rotate around the hinge axis to move close to each other respective end portions (opposite to the hinge axis).

The first clamp 40 comprises a clamping screw 40a active on both the first clamp portion 41 and a second clamp portion 42 for moving close to each other the end portions of the first clamp portion 41 and a second clamp portion 42.

By putting the first clamp 40 on the first flange 29 and on the second flange 33 and by screwing the clamping screw 40a, the first conical contact surface 43 and the second conical contact surface 44 slide onto the conical surface 31 of the first flange 29 and onto the conical surface 35 of the second flange 33 and the exert forces onto the conical surface 31 of the first flange 29 and onto the conical surface 35 of the second flange 33. Such forces have a radial component that presses the abutting surface 30 of the first flange 29 against the abutting surface 34 of the second flange 33.

By unscrewing the clamping screw 40a, the first flange 29 can be decoupled from the second flange 33 and the inserting portion 36 of the second tubular body 27 can be extracted from the internal cavity 28 of the first tubular body 26.

In the preferred embodiment, the clamping screw 40a is a butterfly screw.

For allowing the coating composition pressurized in the pump chamber 13 to reach nozzles (not illustrated), the pump head 20 comprises a delivery manifold 45 that is in fluid communication with the delivery port 24 and that can be fluidly connected to a delivery pipe 102 (partially illustrated in figures 1 and 2).

The delivery manifold 45 is substantially identical to the inlet manifold 25.

The delivery manifold 45 is substantially realized in two dismountable parts. Particularly, the delivery manifold 45 comprises a first tubular body 46 and a second tubular body 47.

The first tubular body 46 is connected to the pump head 20 preferably permanently. In the preferred embodiment, the first tubular body 46 is integral with the pump head 20. The first tubular body 46 comprises an internal cavity 48 that extends along the whole first tubular body 46 and that is in fluid communication with the delivery port 24.

At an outer radial end of the first tubular body 46, there is provided a first flange 49 having an abutting surface 50. The first flange 49 is integral with the first tubular body 46. The abutting surface 50 is a planar surface of annular shape. At the abutting surface 50 the internal cavity 48 is open in a radially outer direction. The first flange 49 further comprises a conical surface 51 radially opposite to the abutting surface 50. The first flange 49 is tapered at the conical surface 51 so as to link the abutting surface 50 to a lateral wall of the first tubular body. The first flange 49 is tapered at the conical surface 51 so as to progressively increase its transversal section in a radial direction directed toward the abutting surface 50. The abutting surface 50 is radially outer with respect to the conical surface 51 of the first flange 49. The conical surface 51 of the first flange 49 is radially interposed between the delivery port 24 and the abutting surface 50.

The second tubular body 47 comprises an internal cavity 52 that extends along the whole second tubular body 47 from a radially inner end to a radially outer end. The radially outer end of the second tubular body 47 can be fluidly connected to the delivery pipe 102 for example by a compression fitting 103.

The second tubular body 47 comprises a second flange 53 having an abutting surface 54. The second flange 53 is integral with the second tubular body 47. The second flange 53 is provided along the second tubular body 47 at a position between the radially inner end and the radially outer end of the second tubular body 47. The abutting surface 54 is a planar surface of annular shape and presents the same dimension of the abutting surface 50 of the first flange 49. The abutting surface 54 is countershaped to the abutting surface 50 of the first flange 49. The second flange 53 along with the abutting surface 54 is crossed by the internal cavity 52. The second flange 53 further comprises a conical surface 55 radially opposite to the abutting surface 54. The second flange 53 is tapered at the conical surface 55 so as to link the abutting surface 54 to a lateral wall of the second tubular body. The second flange 53 is tapered at the conical surface 55 so as to progressively increase its transversal section in a radial direction directed toward the abutting surface 54. The abutting surface 54 is radially inner with respect to the conical surface 55 of the second flange 53. The abutting surface 54 is radially interposed between the delivery port 24 and the conical surface 55 of the second flange 53.

The abutting surface 34 of the second flange 33 is coupled to the abutting surface 30 of the first flange 29.

The second tubular body 47 comprises an insertion portion 56 slidingly inserted into the internal cavity 48 of the first tubular body 46 and in fluid-tight connection with the delivery port 24. The insertion portion 56 has a transversal dimension (namely a dimension along a plane perpendicular to the redial extension of the insertion portion 56) that is slightly smaller that a transversal dimension of the internal cavity 48 of the first tubular body 46.

The insertion portion 56 substantially extends from the abutting surface 54 to the delivery port 24. Particularly, a gasket seal 58 is interposed between the insertion portion 56 and the delivery port 24 so as to fluidly couple the internal cavity 52 to the delivery port 24. The gasket seal 58 is partially inserted into a ring groove 59 (figure 5) provided at a radially inner end of the second tubular body 47 that coincides with a radially inner end of the insertion portion 56. The ring groove 59 holds the gasket seal 58 to the second tubular body 47.

As schematically illustrated in figure 5, a distance L4 in a radial direction between the abutting surface 50 of the first flange 49 and the delivery port 24 is substantially equal to the sum of length L5 of the insertion portion 56 and a length L6 of a portion of the gasket seal 58 extending radially beyond the ring groove 59. The length L5 of the insertion portion 56 is measured from the abutting surface 54 of the second flange 53 and the inner end of the insertion portion 56.

In order to ensure a fluid tight coupling between the abutment surface 50 of the first flange 49 and the abutting surface 54 of the second flange 53 and thus a fluid tight coupling between the internal cavities 48, 52 of the first tubular body 46 and of the second tubular body 47, the abutment surface 50 of the first flange 49 and the abutting surface 54 of the second flange 53 are pressed to each other.

In this connection, the pump assembly 10 comprises a second clamp 60 active on the first flange 49 and on the second flange 53.

The second clamp 60 comprises a first clamp portion 61 and a second clamp portion 62 hinged to each other along a hinge axis radially oriented. The second clamp 60 comprises a first conical contact surface 63 and a second conical contact surface 64.

The first conical contact surface 63 extends on both the first clamp portion 61 and a second clamp portion 62, as well as the second conical contact surface 64 extends on both the first clamp portion 61 and a second clamp portion 62. In other words, the first clamp portion 61 comprises a first part of the first conical contact surface 63 and of the second conical contact surface 64 and the second clamp portion 62 comprises a second part of the first conical contact surface 63 and of the second conical contact surface 64.

The second clamp 60 is configured for clamping the first flange 49 and the second flange 53 to each other so as to couple the first tubular body 46 to the second tubular body 47.

To this end, the first conical contact surface 63 faces the conical surface 51 of the first flange 49 and the second conical contact surface 64 faces the conical surface 55 of the second flange 53. The first conical contact surface 63 is countershaped to the conical surface 51 of the first flange 49 and the second conical contact surface 64 is countershaped the conical surface 55 of the second flange 53.

The first clamp portion 61 and a second clamp portion 62 can rotate around the hinge axis to move close to each other respective end portions (opposite to the hinge axis).

The second clamp 60 comprises a clamping screw 60a active on both the first clamp portion 61 and a second clamp portion 62 for moving close to each other the end portions of the first clamp portion 61 and a second clamp portion 62.

By putting the second clamp 60 on the first flange 49 and on the second flange 53 and by screwing the clamping screw 60a, the first conical contact surface 63 and the second conical contact surface 64 slide onto the conical surface 51 of the first flange 49 and onto the conical surface 55 of the second flange 53 and the exert forces onto the conical surface 51 of the first flange 49 and onto the conical surface 55 of the second flange 53. Such forces have a radial component that presses the abutting surface 50 of the first flange 49 against the abutting surface 54 of the second flange 53.

By unscrewing the clamping screw 60a, the first flange 49 can be decoupled from the second flange 53 and the inserting portion 56 of the second tubular body 47 can be extracted from the internal cavity 48 of the first tubular body 46.

In the preferred embodiment, the clamping screw 60a is a butterfly screw.

The pump assembly 10 comprises a connection interface 66 for allowing a quick mounting and dismounting of the pump head 20 from the pump casing 11.

The connection interface 66 is axially interposed between the pump head 22 and the inspection casing 17.

The connection interface 66 comprises a plurality of through slots 67 wherein each through slot 67 presents an arched shape extending in a circumferential direction. Each through slot 67 comprises a first portion 68 and a second portion 69. The first portion 68 has an extension in a radial direction smaller than the extension in radial direction of the second portion 69. The first portion 68 and the second portion 69 are circumferentially adjacent.

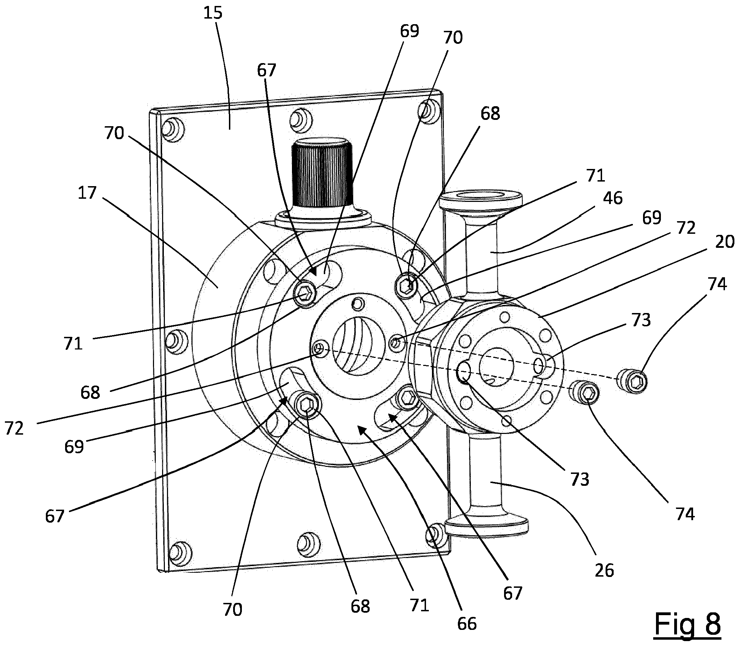

It is provided a first plurality of bolts 70 (figure 8), in particular a bolt 70 for each through slot 67. Each bolt 70 is screwed into a threaded hole (not shown) provided in the inspection casing 17. Each bolt 70 comprises a bolt head 71 having an extension in radial direction greater than the radial extension in radial direction of the first portion 68 and smaller than the radial extension in radial direction of the second portion 69 of a corresponding through slot 67. The connection interface 66 can be rotated about the rotational axis X so as to simultaneously axially align the bolt head 71 of each bolt 70 to the first portion 68 or to the second portion 69 of the corresponding through slot 67. When the bolt heads 71 are aligned to the second portion 69 of the through slots 67, the connection interface 66 can be axially moved with respect to the inspection casing 17 and dismounted from the pump casing 11. When the bolt heads 71 are aligned to the first portion 69 of the through slots 67, the connection interface 66 cannot be axially moved with respect to the inspection casing 17 and, by tightening the bolts 70, the connection interface 66 can be stably coupled to the inspection casing 17 also preventing any rotation of the connection interface 66 around the rotational axis X.

The connection interface 66 further comprises a plurality of threaded holes 72 (figure 8) facing the head pump 20. The head pump 20 comprises a plurality of through holes 73 axially aligned to the plurality of threaded holes 72. A second plurality of bolts 74 engages the through holes 73 and is screwed into the plurality of threaded holes 72, so as to stably connect the pump head 20 to the connection interface 66.

The head pump 66 can be this mounted and dismounted from the pump casing 11 without completely unscrewing any bolt from its seat.

In the preferred embodiment of the invention, the connection interface 66 is completely made of Polyether ether ketone (PEEK) so as to withstand high temperature and to reduce the friction with the inspection casing 17 during its rotation.

The pump assembly 10 comprises a first electric heater 75, a second electric heater 76 and a third electric heater 77.

All the electric heaters 75, 76, 77 can be cartridge heaters. All the electric heaters 75, 76, 77 are enclosed in a cylindrical container or have a cylindrical shape. The first electric heater 75, the second electric heater 76 and the third electric heater 77 are active into the pump head 22 in the proximity of the pump chamber 13.

To this end, the first electric heater 75, the second electric heater 76 and the third electric heater 77 are inserted into receiving holes 78 (schematically illustrated in figure 7) provided in the pump head 20. The receiving holes 78 are axially oriented.

The pump assembly 10 further comprises a thermal probe 79 active within the pump head 20 for sensing the temperature of the pump head 20 at least close to the pump chamber 13.

The thermal probe 79 can be a thermistor. The thermal probe 79 is enclosed in a cylindrical container or has a cylindrical shape. The pump head 20 comprises a receiving hole 78 for the thermal probe 79 too.

The pump assembly 10 comprises a holding ring 80 for stably connecting the first electric heater 75, the second electric heater 76, the third electric heater 77 and the thermal probe 79 to the pump head 20.

The holding ring 80 is mechanically connected to the pump head 20 in a position axially opposite to the pump casing 11. The holding ring 80 is mechanically connected to the pump head 20 though two butterfly screws 81 (figure 7) screwed into threaded holes (not illustrated) provided in the head pump 20. The two butterfly screws 81 act along an axial direction and are substantially parallel to the first electric heater 75, the second electric heater 76, the third electric heater 77 and the thermal probe 79.

The holding ring 80 comprises through holes 82 each of which is stably engaged by one of the first electric heater 75, the second electric heater 76, the third electric heater 77 and the thermal probe 79.

By unscrewing the butterfly screws 82, the holding ring 80 along with the first electric heater 75, the second electric heater 76, the third electric heater 77 and the thermal probe 79 can be simultaneously dismounted from the head pump 20.

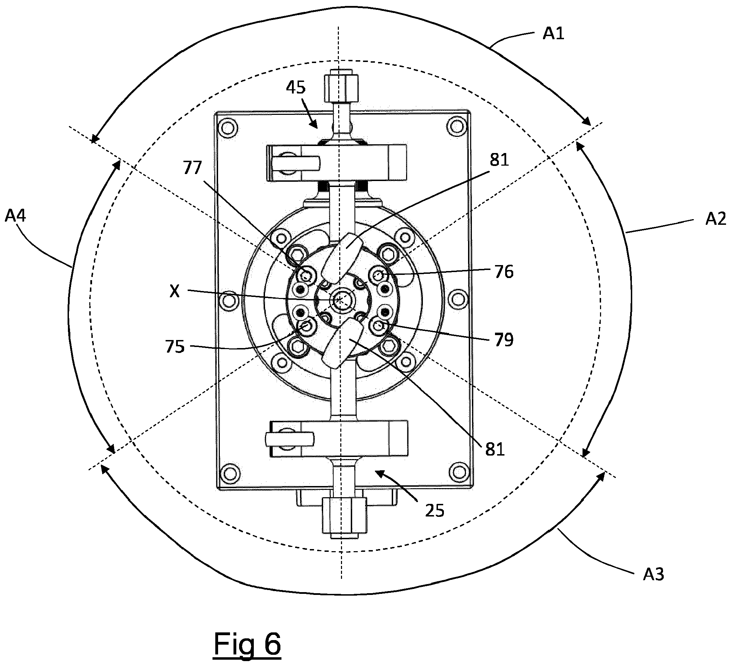

As schematically illustrated in figure 6, the first electric heater 75, the second electric heater 76, the third electric heater 77 and the thermal probe 79 are positioned circumferentially around the rotational axis X on the pump head 20. The butterfly screws 82 are positioned circumferentially around the rotational axis X on the pump head 20 too.

In the preferred embodiment, the first electric heater 75, the second electric heater 76, the third electric heater 77 and the thermal probe 79 are spaced apart in the circumferential direction by respective angles of about 60° or of about 120°.

In particular, the third electric heater 77 and the second electric heater 76 are spaced apart in the circumferential direction by an angle A1 of about 120°, the second electric heater 76 and the thermal probe 79 are spaced apart in the circumferential direction by an angle A2 of about 60°, the thermal probe 79 and the first electric heater 75 are spaced apart in the circumferential direction by an angle A3 of about 120°, and the first electric heater 75 and the third electric heater 77 are spaced apart in the circumferential direction by an angle A4 of about 60°.

The butterfly screws 82 are respectively circumferentially interposed between the second electric heater 76 and the third electric heater 77 and between the thermal probe 79 and the first electric heater 75. In particular, the butterfly screw 82 interposed between the second electric heater 76 and the third electric heater 77 is spaced apart in the circumferential direction from the second electric heater 76 by an angle of about 60°. The butterfly screw 82 interposed between the first electric heater 75 and the thermal probe 79 is spaced apart in the circumferential direction from the first electric heater 75 by an angle of about 60°. As a results, the butterfly screws 82, the first electric heater 75, the second electric heater 76, the third electric heater 77 and the thermal probe 79 are evenly distributed in the circumferential direction on the pump head 20.

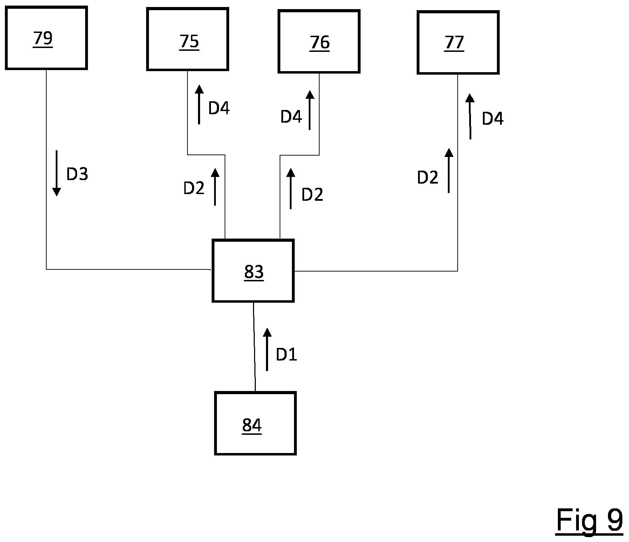

The first electric heater 75, the second electric heater 76, the third electric heater 77 and the thermal probe 79 are in signal communication with a control unit 83 (schematically illustrated in figure 9).

In a possible embodiment, the control unit 83 is configured to receive a first input data D1 representative of a settled temperature or of a settled range of temperature. The first input data D1 can be generated by a user interface 84 where a user sets a temperature or a range of temperature.

The control unit 83 is configured for generating a second input data D2 suitable for activating one or more of the first electric heater 75, the second electric heater 76 and the third electric heater 77.

The control unit 83 is configured to receive a third input data D3 from the thermal probe 79 representative of a temperature sensed by the thermal probe 79.

The control unit 83 is configured to compare the first input data D1 and the third input data D3. When the third input data D3 is lower than the first input data D1, the control unit 83 generates the second input data D2 for activating one or more of the first electric heater 75, the second electric heater 76 and the third electric heater 77. When the third input data D3 is higher than the first input data D1, the control unit 83 generates fourth input data D4 suitable for deactivating one or more of the first electric heater 75, the second electric heater 76 and the third electric heater 77.

The pump head 20, the inlet manifold 26 and the delivery manifold 45 are made of stainless steel. The inspection casing 17 and preferably the whole pump casing 11 are made of stainless steel. The pump chamber is made of stainless steel. The connection interface 66 and the holding ring 80 are made of Polyether ether ketone (PEEK).

## Claims

1. Pump assembly (10) for the treatment of injection medical devices comprising:
a pump casing (11) housing a driver connected to a pumping body (12) rotating around a rotational axis (X);
a pump head (20) comprising a pump chamber (13), wherein the pumping body (12) acts into said pump chamber (13) for pressurizing a coating composition, an inlet port (23) for a coating composition and a delivery port (24) for said coating composition, wherein said inlet port (23) and said delivery port (24) are in fluid connection with said pump chamber (13);
an inlet manifold (25) for entering said coating composition into said inlet port (23) and a delivery manifold (45) for exiting said coating composition from said delivery port (24);
wherein both the inlet manifold (25) and the delivery manifold (45) comprise respectively:
a first tubular body (26, 46) connected to the pump head (20) and comprising an internal cavity (28, 48) facing respectively the inlet port (23) and the delivery port (24), and a first flange (29, 49) comprising an abutting surface (30, 50);
a second tubular body (27, 47) having an insertion portion (36, 56) inserted into the internal cavity (28, 48) of the first tubular body (26, 46) and in fluid-tight connection respectively with the inlet port (23) and the delivery port (24), and a second flange (33, 53) provided at a position between a radially inner end and a radially outer end of the second tubular body (27, 47) and comprising an abutting surface (34, 54) sets against the abutting surface (30, 50) of the first flange (29, 49);
the pump assembly further comprising a first clamp (40) active on the first flange (29) and the second flange (33) of the inlet manifold (25) to press the abutting surface (34) of the second flange (33) against the abutting surface (30) of the first flange (29) and a second clamp (60) active on the first flange (49) and the second flange (53) of the delivery manifold (45) to press the abutting surface (54) of the second flange (53) against the abutting surface (50) of the first flange (49).

2. Pump assembly (10) according to claim 1, wherein the insertion portions (36, 56) of the second tubular body (27, 47) of the inlet manifold (25) and of the delivery manifold (45) extend from the respective abutting surface (30, 50) of the first flange (29, 49) to a gasket seal (38, 58) located respectively at the inlet port (23) and at the delivery port (24).

3. Pump assembly (10) according to claim 1 or 2, wherein the first flange (29) and the second flange (33) of the inlet manifold (25) comprise respective conical surfaces (31, 35) opposite to the abutting surfaces (30, 34) and wherein said first clamp (40) comprises a first conical contact surface (43), a second conical contact surface (44) and a clamping screw (40a) for slidingly coupling the conical contact surfaces (43, 44) to the respective conical surfaces (31, 35) of the first flange (29) and of the second flange (33); wherein the first flange (49) and the second flange (53) of the delivery manifold (45) comprise respective conical surfaces (51, 55) opposite to the abutting surfaces (50, 54) and wherein said second clamp (60) comprises a first conical contact surface (63), a second conical contact surface (64) and a clamping screw (60a) for slidingly coupling the conical contact surfaces (63, 64) to the respective conical surfaces (51, 55) of the first flange (49) and of the second flange (53).

4. Pump assembly (10) according to any of the preceding claims, comprising a first electric heater (75) and a second electric heater (76) active in the pump head (20) at the pump chamber (13) and configured to heat said pump head (20).

5. Pump assembly (10) according to claim 4, comprising a third electric heater (77) and a thermal probe (79) active in the pump head (20) at the pump chamber (13), wherein the third electric heater (77) is configured to heat said pump head (20) and wherein said thermal probe (79) is configured to sensing a temperature of the pump head (20) at the pump chamber (13); said third electric heater (77) being circumferentially interposed between said first electric heater (75) and said second electric heater (76), said second electric heater (76) being circumferentially interposed between said third electric heater (77) and said thermal probe (79), said thermal probe (79) being circumferentially interposed between said first electric heater (75) and said second electric heater (76).

6. Pump assembly (10) according to claim 4 or 5, wherein the first electric heater (75) and the second electric heater (76) are spaced apart from each other in a circumferential direction by an angle comprised between 90° and 180°.

7. Pump assembly (10) according to claim 5, wherein any two circumferentially adjacent elements selected from group consisting of:
first electric heater (75), second electric heater (76), third electric heater (77) and thermal probe (79), are spaced apart from each other in the circumferential direction by angles comprised between 60° and 120°.

8. Pump assembly (10) according to any claim from 5 to 7, wherein said thermal probe (79), said first electric heater (75), said second electric heater (76) and said third electric heater (77) are in signal communication with a control unit (83); said control unit (83) being configured to maintain the pump head (30) temperature within a predetermined temperature range.

9. Pump assembly (10) according to any of the preceding claims, comprising a connection interface (66) between said pump head (20) and said pump casing (11), said connection interface (66) being mechanically connected to said pump casing (11) through a first plurality of bolts (70) and said pump head (20) being connected to said connection interface (66) through a second plurality of bolts (74) different from said first plurality of bolts (70).

10. Pump assembly (10) according to claim 9, wherein said connection interface (66) comprises a through slot (67) for each bolt (70) of said first plurality of bolts (70), wherein each through slot (67) comprises a first portion (68) configured to be contacted by a bolt head (81) to axially retain the connection interface (66) on the pump casing (11) and a second portion (69), circumferentially adjacent to the first portion (68), configured to be traversed by the bolt head (81).
